# EUROPÄISCHE PATENTSCHRIFT

(11) **EP 3 077 066 B1**
(45) Veröffentlichungstag und Bekanntmachung des Hinweises auf die Patenterteilung: **28.04.2021**
(21) Anmeldenummer: 14833106.9
(22) Anmeldetag: 04.12.2014
(51) Int. Cl.: A63B 23/18, A61B 5/00

(54) **VERFAHREN UND VORRICHTUNG ZUR ERSTELLUNG UND AUSGABE EINES REIZES UND/ODER EINER ATEMAUFFORDERUNG FÜR EINEN MENSCHEN UND/ODER EIN TIER**
METHOD AND DEVICE FOR CREATING AND OUTPUTTING A STIMULUS AND/OR AN INSTRUCTION TO BREATHE FOR A PERSON AND/OR AN ANIMAL
PROCÉDÉ ET DISPOSITIF POUR GÉNÉRER ET ÉMETTRE UNE STIMULATION ET/OU UN ORDRE DE RESPIRATION POUR UN ÊTRE HUMAIN ET/OU UN ANIMAL

(30) Priorität: 04.12.2013 AT 9362013
(43) Veröffentlichungstag der Anmeldung: 12.10.2016
(73) Patentinhaber: Human Research Institut für Gesundheitstechnologie und Präventionsforschung GmbH, 8160 Weiz (AT); Joysys GmbH, 8160 Weiz (AT)
(72) Erfinder: FRÜHWIRTH, Matthias, 8042 Graz (AT); HASSLER, Thomas, 8045 Graz (AT); MESSERSCHMIDT, Dietmar, 8051 Graz (AT); MOSER, Maximilian, 9074 Keutschach (AT); PUSWALD, Bernhard, 8046 Stattegg (AT)
(74) Vertreter: Gibler & Poth Patentanwälte KG
(86) Internationale Anmeldenummer: PCT/AT2014/000216
(87) Internationale Veröffentlichungsnummer: WO 2015/081355

(56) Entgegenhaltungen:
- WO-A1-2009/147599
- WO-A1-2011/045709
- US-A1- 2005 096 555

## Beschreibung

Die Erfindung betrifft eine Vorrichtung zur Erstellung und Ausgabe eines Reizes und/oder einer Atemaufforderung für einen Menschen und/oder ein Tier gemäß dem Gattungsbegriff des Patentanspruches 1.

Die Herzratenvariabilität bezeichnet die Schwankungen der Herzrate eines Menschen. Diese wird von zahlreichen Faktoren beeinflusst. Es ist bekannt, dass zwischen Atmung und der Herzratenvariabilität bei positiven emotionalen Zuständen ein Zusammenhang besteht, welcher unter der Bezeichnung "respiratorische Sinusarrhytmie" beschrieben wurde. Dieser Zusammenhang bricht bei Stress oder anderen Belastungen zusammen.

Derzeit sind verschiedene Methoden bekannt, welche darauf zielen die Herzratenvariabilität zu erhöhen und einen Zustand entsprechend der respiratorischen Sinusarrhytmie zu verursachen, da es sich gezeigt hat, dass dies etwa den individuellen Stresspegel senkt, und so ein individuelles körperliches Wohlbefinden fördert. Derartige bekannte Verfahren arbeiten dabei mit, von dem betreffenden Probanden als eindeutig positiv empfundene Zustände zu verursachen, um derart die Herzratenvariabilität zu erhöhen. Diese Methoden sind jedoch hinsichtlich deren Wirksamkeit von der Beziehung des Probanden zu einem Betreuer bzw. Berater, sowie den individuellen Fähigkeiten des Betreuers bzw. Beraters selbst abhängig, und nur schwer vorhersagbar bzw. reproduzierbar.

Die US 2005/096555 A1 beschreibt ein Verfahren zur Anpassung bzw. Synchronisation der Atmung und des Herzschlages um eine maximale Kohärenz zur Herzratenvariabilität zu erlangen. Dabei ist vorgesehen, dass eine Atemaufforderungen jeweils im Bereich der Wendepunkte der Herzratenvariabilität ausgegeben werden.

Die WO 2011/045709 A1 beschreibt ein System zur Atemsteuerung, um die Herzratenvariabilität zu verändern. Dabei wird ein taktiles Signal über einen Zeitraum von mehreren Herzschlägen ausgegeben.

Die WO 2009/147599 A1 beschreibt ebenfalls ein System zur Beeinflussung der Herzratenvariabilität durch gezieltes Atmen. Dabei wird eine Atemaufforderung über mehrere Herzschläge ausgegeben, wobei Anfang und Ende der Atemaufforderung jeweils beim Auftreten einer R-Zacke erfolgen.

Aufgabe der Erfindung ist es daher ein Verfahren der eingangs genannten Art anzugeben, mit welchem die genannten Nachteile vermieden werden können, und mit welchem gezielt die Herzratenvariabilität beeinflusst werden kann.

Erfindungsgemäß wird dies durch die Merkmale des Patentanspruches 1 erreicht.

Dadurch kann die Herzratenvariabilität gezielt und effektiv trainiert werden, wobei dieses Training ausschließlich auf körperlich messbaren Effekten beruht. Es hat sich dabei gezeigt, dass durch die gegenständlich beschriebene Vorgabe von Reizen bzw. Atemaufforderungen eine gezielte Beeinflussung der Herzratenvariabilität eines Probanden erreicht werden kann. Dadurch kann gezielt ein bestimmter Zustand eines Probanden hervorgerufen werden, welcher sich sowohl physisch wie auch psychisch auf diesen Auswirkt. Durch geeignete Auswahl des ersten Zeitpunkts in dem Reiz-Zeitbereich, wie auch der Art des Reizes kann vorgebbar ein bestimmter Zustand bei einem Probanden verursacht werden. So kann etwa bei Ausgabe eines Einatmenzeitpunkts innerhalb der sog. elektrischen Diastole und Einatmen des Probanden zu diesen Zeitpunkten eine erhebliche Steigerung der Herzratenvariabilität erzielt werden kann. Dadurch kann das Wohlbefinden und die Leistungsfähigkeit eines Probanden nachhaltig und einfach gesteigert werden. Dadurch kann der Gesundheitszustand eines Probanden verbessert werden. Dadurch kann die Steigerung der Herzratenvariabilität auf Basis messbarer Effekte erfolgen, losgelöst von esoterischen Einflüssen, wie diese etwa bei zahlreichen Lehrgängen zu diesem Thema nach wie vor feststellbar sind. Dabei ist ein Trainingseffekt messbar nachvollziehbar. Dadurch kann ein Zeitpunkt des Atmens messtechnisch begründet vorgegeben werden, unabhängig von nicht reproduzierbaren Einflüssen, wie etwa Gefühlen, Intuition oder einer sog. Tagesverfassung. Indem der Proband gezielt Reizen ausgesetzt wird, kann ein solcher Effekt zudem ohne aktive Mitwirkung des Probanden erreicht werden. Alternativ kann jedoch auch gezielt die Herzratenvariabilität gesenkt werden, wenn bei einem Probanden eine zu hohe Herzratenvariabilität vorliegt. Dies kann etwa Menschen die unter einem Burnout-Syndrom leiden, oder bei Wachkomapatienten der Fall sein.

Die Unteransprüche betreffen weitere vorteilhafte Ausgestaltungen der Erfindung.

Die Erfindung betrifft eine Vorrichtung zur Erstellung und Ausgabe eines Reizes und/oder einer Atemaufforderung für einen Menschen und/oder ein Tier.

Aufgabe der Erfindung ist es daher eine Vorrichtung der vorstehend genannten Art anzugeben, mit welcher die eingangs genannten Nachteile vermieden werden können, und mit welcher gezielt die Herzratenvariabilität beeinflusst werden kann.

Dadurch können die vorstehend zum Verfahren dargelegten Vorteilhaften Wirkungen erzielt werden.

Ausdrücklich wird hiermit auf den Wortlaut der Patentansprüche Bezug genommen, wodurch die Ansprüche an dieser Stelle durch Bezugnahme in die Beschreibung eingefügt sind und als wörtlich wiedergegeben gelten.

Die Erfindung wird unter Bezugnahme auf die beigeschlossenen Zeichnungen, in welchen lediglich bevorzugte Ausführungsformen beispielhaft dargestellt sind, näher beschrieben. Dabei zeigt:
Fig. 1 ein Blockschaltbild einer bevorzugten Ausführungsform einer gegenständlichen Vorrichtung;
Fig. 2 ein Beispiel eines EKGs;
Fig. 3 einen zeitlichen Verlauf der Herzrate; und
Fig. 4 ein Detail der Ansicht gemäß Fig. 2.

Die gegenständliche Erfindung betrifft eine Vorrichtung 11 sowie ein Verfahren zur Ermittlung und Ausgabe eines Reizes und/oder einer Atemaufforderung. Dabei ist vorgesehen, dass Messungen zumindest einer Körperfunktion eines Menschen bzw. eines Tieres durchgeführt werden, und dass aufgrund dieser Messungen ein Signal erzeugt und ausgegeben bzw. angezeigt wird. Dieses Signal stellt entweder einen Reiz dar oder eine Atemaufforderung, daher eine Aufforderung an den Probanden einzuatmen bzw. auszuatmen. Im Gegensatz zu einem Reiz, welcher unwillkürlich auf den Probanden wirkt, steht es diesem bei einer Atemaufforderung, es kann sich dabei sowohl um eine Einatemaufforderung, wie auch um eine Ausatemaufforderung handeln, selbst frei dieser Aufforderung zu folgen.

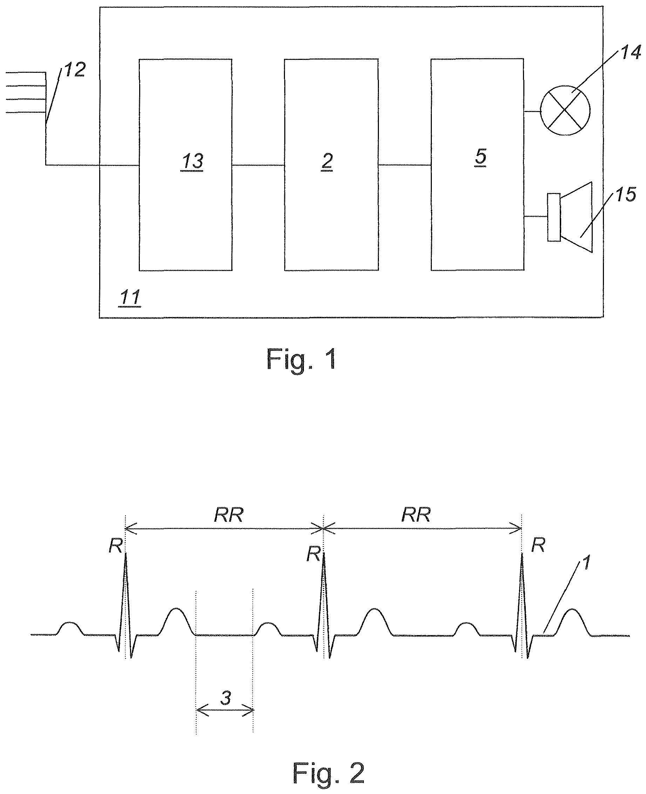

Fig. 1 zeigt dabei ein Blockschaltbild einer Vorrichtung 11 zur Ermittlung und Ausgabe eines Reizes bzw. einer Atemaufforderung, wobei die Vorrichtung 11 eine, als EKG-Einheit 13 ausgebildete, Herzsignalermittlungseinheit, eine Auswerteeinheit 2 und eine Signaleinheit 5 aufweist. EKG 1 ist dabei die übliche Abkürzung für Elektrokardiogramm. Bevorzugt ist vorgesehen, dass die Herzsignalermittlungseinheit als EKG-Einheit 13 ausgebildet ist. Im Weiteren wird die Erfindung hinsichtlich eines EKGs und einer EKG-Einheit 13 beschrieben, was jedoch bevorzugt nicht einschränkend auszulegen ist. Es hat sich für einfache Implementierungen als ausreichend erwiesen, lediglich einen sog. Arterienpuls zu detektieren. Es kann als Herzsignal auch mechanisches Herzsignal, etwa ein Balistokardiogramm und/oder ein Seismokardigramm, verwendet werden. Bevorzugt wird eine zeitgenaue Detektion der R-Zacke als ausreichend erachtet.

Weiters wird nachfolgend der Begriff Proband verwendet, was bevorzugt sowohl tierische wie auch menschliche Probanden mit einschließt.

Bei der EKG-Einheit 13 kann es sich um jede Art eines EKG-Geräts bzw. einer Vorrichtung zur Erstellung eines EKGs 1 handeln, wobei insbesondere vorgesehen ist, dass die betreffende EKG-Einheit 13 eine digitale EKG-Einheit 13 mit einer hohen Zeitauflösung und/oder einer hohen Samplingrate, von beispielsweise 8 kHz, ist. Durch die hohe Auflösung kann die R-Zacke des EKGs 1 besser detektiert werden, wodurch eine genaue Ermittlung der Herzrate RR sowie daraus folgend der Herzratenvariabilität HRV möglich ist. Die EKG-Einheit 13 ist mit Eingängen für EKG-Kabel 12 schaltungstechnisch verbunden.

Die EKG-Einheit 13 ist weiters schaltungstechnisch mit der Auswerteeinheit 2 verbunden. Bei der Auswerteeinheit 2 handelt es sich bevorzugt um ein Mikrocomputersystem bzw. ein sog. Embedded System, welches bevorzugt einen Mikrocontroller umfasst. Die Auswerteeinheit 2 ist ausgebildet, zufolge des nachfolgend beschriebenen Verfahrens einen Reiz und/oder eine Atemaufforderung zu erstellen bzw. zu ermitteln. Es kann auch vorgesehen sein, die nachfolgend beschriebenen Funktionalitäten in Form eines ASIC auszubilden.

Die Auswerteeinheit 2 ist mit der Signaleinheit 5 verbunden, welche vorgesehen und ausgebildet ist, nach Ansteuerung durch die Auswerteeinheit 2 einen Reiz bzw. eine Atemaufforderung in Form eines Signals zu erzeugen und auszugeben.

Die Ausgabe des Reizes bzw. der Atemaufforderung ist insbesondere an ein Signalmittel vorgesehen, etwa ein Leuchtmittel 14, wie beispielsweise eine LED, und/oder ein Lautsprecher 15. Entsprechend ist die Signaleinheit 5 bevorzugt umfassend einen Analogverstärker ausgebildet. Es kann auch vorgesehen sein, alternativ, optional und/oder zusätzlich hiezu, den Reiz bzw. die Atemaufforderung in Form eines Datenpaktes auszugeben, wobei hiezu vorgesehen ist, dass die Signaleinheit 5 eine Schnittstelle, etwa einen USB-Port oder eine drahtlose Schnittstelle, wie etwa Bluetooth, aufweist, sowie die entsprechenden Treiberschaltungen bzw. Controller aufweist.

Hinsichtlich der Ausgabe der Atemaufforderung ist lediglich relevant, dass diese durch den Probanden als solche erkennt und verstanden wird. Relevant dabei ist lediglich dass es sich um ein klar und eindeutig identifizierbares Signal handelt. Dies kann je nach Region und Kulturkreis unterschiedlich bevorzugt sein.

Gemäß einer bevorzugten Ausführungsform ist vorgesehen, dass, bei Ausgabe einer Atemaufforderung überprüft wird, ob und/oder nach welcher Atemlatenz-Zeit nach der Abgabe der Atemaufforderung der Mensch und/oder das Tier atmet. Dies kann etwa mittels eines entsprechend mit Messgeräten, etwa Dehnmessstreifen ausgestatteten, Brustgurtes überprüft werden, welcher die Kontraktion und Expansion des Brustkorbes aufnimmt und mit der Auswerteinheit in Kommunikationsverbindung steht.

Derart kann eine Atemlatenz-Zeit ermittelt werden, daher eine Zeitspanne, welche vom Absetzen der Atemaufforderung bis zur tatsächlichen Durchführung des Atemvorganges durch den Probanden vergeht. Diese Atemlatenz-Zeit kann nachfolgend bei der Abgabe der Atemaufforderung berücksichtigt werden, indem etwa der erste Zeitpunkt 4 um die Atemlatenz-Zeit korrigiert wird.

Ein Reiz wirkt bereits unbewusst lediglich dadurch, dass der Proband diesem Reiz ausgesetzt ist bzw. wird, wobei lediglich relevant ist, dass der betreffende Reiz durch den Probanden wahrgenommen wird. Hiezu kann eine Überwachung einzelner Sinnesbereiche des Probanden vorgesehen sein.

Gemäß einer ersten bevorzugten Ausführungsform ist vorgesehen, dass der, als Signal ausgegebene Reiz ein optischer Reiz, insbesondere ein Lichtreiz, ist, und dass das Signal als optisches Signal ausgegeben wird. Dabei ist insbesondere vorgesehen, dass ein vorgebbarer Farbwechsel und/oder Helligkeitswechsel als Reiz verwendet wird. Ein derartiger optischer Reiz kann etwa mittels eines Monitors ausgegeben werden. Bevorzugt ist etwa ein Wechsel der Lichtfarbe, welcher der Proband ausgesetzt ist, von Rot zu Blau und/oder Grün vorgesehen.

Gemäß einer zweiten bevorzugten Ausführungsform ist vorgesehen, dass der, als Signal ausgegebene Reiz ein akustischer Reiz ist, und dass das Signal als akustisches Signal ausgegeben wird. Dabei ist insbesondere vorgesehen, dass ein Ton, ein Klang oder ein Geräusch ausgegeben wird, wobei vorgebbare Wechsel zwischen Tönen, Geräuschen und/oder Klängen unterschiedlicher Lautstärke, Klangfärbung, Grundtonhöhe usw. vorgesehen sein können. Auch ein Wechsel zwischen einem Geräusch und einem Klang kann vorgesehen sein.

Gemäß einer dritten bevorzugten Ausführungsform ist vorgesehen, dass der, als Signal ausgegebene Reiz ein taktiler Reiz ist, und dass das Signal als mechanisches Signal ausgegeben wird. Dabei ist insbesondere vorgesehen, dass der Proband einem vorgebbaren Vibrationswechsel ausgesetzt wird, oder beispielsweise vorgebbar abwechselnd berührt wird.

Gemäß einer vierten bevorzugten Ausführungsform ist vorgesehen, dass der, als Signal ausgegebene Reiz ein olfaktorischer Reiz ist, und dass das Signal als Freisetzung wenigstens eines Duftstoffes ausgegeben wird. Dabei ist insbesondere vorgesehen, dass im Bereich der Nase des Probanden vorgebbar abwechselnd unterschiedliche Duftstoffe in kleinen Mengen freigesetzt, und vorzugsweise nachfolgend jeweils abgesaugt werden.

Jeweils kann vorgesehen sein, durch geeignete Sensoren, wie etwa eine Augenkamera, zu überprüfen ob der Proband in der Lage ist die jeweiligen Reize aufzunehmen. Auch ist bevorzugt vorgesehen, vor Durchführung des Verfahrens jeweils eine Kalibrierung vorzunehmen, welche auch eine grundsätzliche Funktionsüberprüfung des jeweils zu Reizen beabsichtigten Sinn einschließen kann.

Bei dem Verfahren zur Erstellung und Ausgabe eines Reizes und/oder einer Atemaufforderung für einen Menschen und/oder ein Tier ist vorgesehen, dass ein EKG 1, eines menschlichen oder tierischen Probanden durch ein EKG-Gerät bzw. eine EKG-Einheit 13 einer gegenständlichen Vorrichtung 11 aufgenommen wird. Es ist dabei vorgesehen, dass innerhalb des EKGs 1 bzw. des Herzschlagsignals ein vorgebbarer Reiz-Zeitbereich ermittelt wird, welcher im Bereich zwischen zwei Herzschlägen, etwa zwischen zwei R-Zacken angeordnet ist.

Es kann sich bei dem Reiz-Zeitbereich um jeden Zeitbereich innerhalb eines, einem bestimmten Herzschlag zuordenbaren bzw. zugeordneten Herzschlagsignals handeln. Gemäß der nachfolgend weiter beschriebenen Verfahrens ist vorgesehen, dass der vorgebbare Reiz-Zeitbereich ein Zeitbereich der elektrischen Diastole 3 ist. Die nachfolgende Beschreibung ist darauf gerichtet.

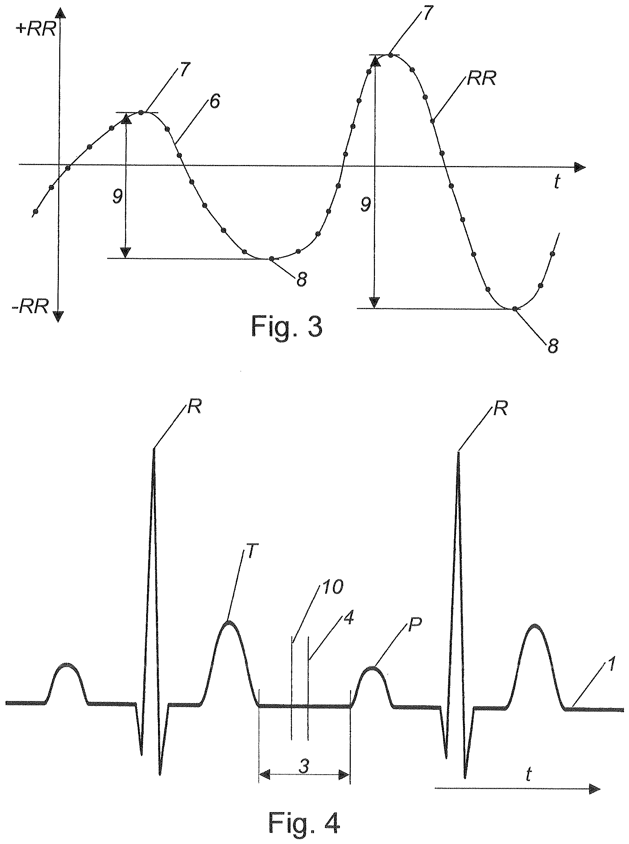

Der Zeitbereich, welcher allgemein als elektrische Diastole 3 bezeichnet wird, liegt zwischen der T-Welle, bzw. sofern vorhanden oder feststellbar der U-Welle, und der nachfolgenden P-Welle. Der betreffende Zeitbereich der elektrischen Diastole 3 ist in den Fig. 2 und 4 eingetragen. Die Ermittlung des Zeitbereichs der elektrischen Diastole 3 erfolgt in der Auswerteeinheit 2, an welche das EKG 1 übermittelt wird. Dabei ist insbesondere vorgesehen, einen Wert für den Zeitbereich der elektrischen Diastole 3 zu ermitteln, indem zumindest der Intervall zwischen zwei R-Zacken ausgewertet wird, vorzugsweise einer vorgebbaren Mehrzahl aufeinander folgender R-Zacken, und danach der derart ermittelte Wert für den Zeitbereich der elektrischen Diastole 3 für die nachfolgenden Herzschläge bzw. R-Zacken ebenfalls verwendet wird. Insbesondere kann vorgesehen sein, den Zeitbereich der elektrischen Diastole 3 durch ein Wertpaar, bestehend aus einer Startzeit und einer Endzeit, welche jeweils von der, der betreffenden elektrischen Diastole 3 vorangehenden R-Zacke an bemessen sind, zu charakterisieren. Eine derartige Charakterisierung kann auch bei anderen gewählten Reiz-Zeitbereichen vorgesehen sein.

Zur Detektion des Bereichs der elektrischen Diastole 3 können verschiedene Verfahren vorgesehen sein, wobei insbesondere vorgesehen ist, die R-Zacken zu detektieren, die Herzrate RR, daher den zeitlichen Abstand zweier aufeinander folgender R-Zacken, zu ermitteln, und eine gewisse Zeit nach einer R-Zacke, welche Zeit von der Herzrate RR abhängig ist, den Zeitbereich der elektrischen Diastole 3 anzunehmen. Dabei handelt es sich um ein approximatives Verfahren, welches jedoch für die gegenständliche Anwendung ausreichend sein kann, da es in einem ersten Schritt ausreichend sein kann, einen Mittenbereich des Zeitbereichs der elektrischen Diastole 3 zu erkennen. Zudem ist dieser bevorzugte Verfahrensschritt einfach und mit lediglich geringen Anforderungen an die Hardware umsetzbar, und unterstützt derart eine mobile Implementierung der gegenständlichen Vorrichtung 11. Es kann auch vorgesehen sein, den betreffenden Zeitbereich der elektrischen Diastole 3 mittels Signalanalyse exakter zu detektieren, was bevorzugt bei stationären Implementierungen des Verfahrens vorgesehen ist.

Die Auswerteeinheit 2 ermittelt einen ersten Zeitpunkt 4 innerhalb des Zeitbereichs der elektrischen Diastole 3, und erzeugt für einen Start-Herzschlag an diesem ersten Zeitpunkt 4 einen Reiz bzw. eine Atemaufforderung, welche an die Signaleinheit 5 übermittelt wird, welche wiederum ein entsprechendes Signal ausgibt. Es ist dabei bevorzugt vorgesehen, dass zwischen den einzelnen beschriebenen Verfahrensschritten lediglich die für die elektronische Verarbeitung erforderlichen Zeiten liegen, und keine Signalpuffer oder dergleichen vorgesehen sind.

Der erste Zeitpunkt 4, welcher auch als Startzeitpunkt bezeichnet werden kann, bezeichnet dabei den Zeitpunkt innerhalb des Zeitbereichs der elektrischen Diastole 3, an welchem bzw. für welchen der Reiz bzw. die Atemaufforderung erstellt bzw. gesendet wird. Der erste Zeitpunkt 4 kann durch dessen zeitlichen Abstand zur R-Zacke eines Herzschlages, insbesondere des Start-Herzschlages, angegeben werden.

Zwischen zwei Atemvorgängen eines Menschen vergehen in der Regel eine gewisse Anzahl an Herzschlägen. Es ist daher vorgesehen, dass von der Auswerteeinheit 2 eine zweite Atemaufforderung an dem ersten Zeitpunkt 4 innerhalb des Reiz-Zeitbereichs bzw. des Zeitbereichs der elektrischen Diastole 3 des Herzschlages, welcher eine vorgebbare Anzahl Herzschläge nach dem ersten Herzschlag nachfolgt, erzeugt und an die Signaleinheit 5 übermittelt wird, und dass die Signaleinheit 5 die zweite Atemaufforderung in Form eines Signals ausgibt. Bevorzugt ist vorgesehen, dass die betreffende Anzahl an Herzschlägen anfangs mit einem Defaultwert von ca. 6 bis 8 vorgegeben ist, welcher nachfolgend angepasst werden kann. Es hat sich dabei gezeigt, dass dies zu einer phasenstarren Kopplung des gegenständlichen Verfahrens mit dem Organismus des Probanden führt.

Dadurch kann die Verfahrensdurchführung auch den gegenwärtigen Zustand des Probanden berücksichtigen, und ist deutlich wirksamer als bekannte Verfahren, welche zwischen zwei Atemaufforderungen eine, vom Organismus des Probanden unabhängige und fest vorgegebene Wartezeit vorschreiben. Dabei hat sich gezeigt, dass ein derartiges Abwarten einer vorgebbaren Anzahl an Herzschlägen des Probanden auch bei der Ausgabe von Reizen einen vorteilhaften Effekt hat, aufgrund besagter Kopplung der Anregung mit dem Organismus des Probanden.

Dabei hat es sich als vorteilhaft erwiesen, dass der zweite Reiz und/oder die zweite Atemaufforderung unterschiedlich von dem ersten Reiz bzw. der ersten Atemaufforderung ist. So kann etwa vorgesehen sein, als erste Atemaufforderung eine Einatemaufforderung auszugeben und als zweite Atemaufforderung eine Ausatemaufforderung auszugeben, oder aber als ersten Reiz den Probanden rotem Licht auszusetzen und als zweiten Reiz blauem Licht. Abhängig von der Art des Reizes oder der Atemaufforderung können auch die Anzahl an Herzschlägen zwischen den beiden Reizen bzw. Atemaufforderungen gemäß dem vorstehenden Absatz variiert werden.

Es kann dabei vorgesehen sein, dass der, vom ersten Reiz unterschiedliche zweite Reiz zu einem anderen Zeitpunkt innerhalb des Reiz-Zeitbereichs ausgegeben wird, als der erste Reiz.

Bevorzugt ist weiters vorgesehen, dass der erste Reiz und/oder der zweite Reiz eine vorgebbare Reiz-Zeitdauer lang ausgegeben werden, wobei die betreffende Reiz-Zeitdauer selbstverständlich kleiner der Zeitdauer besagter Anzahl an Herzschlägen ist. So kann beispielsweise ein erster Reiz in Form einer Vibration mit einer ersten Frequenz ausgegeben werden, dieser erste Reiz beendet werden, und nach einer gewissen Pause ein zweiter Reiz in Form einer zweiten Vibration mit einer zweiten, von der ersten unterschiedlichen Frequenz ausgegeben werden.

Bevorzugt ist vorgesehen, dass der erste Zeitpunkt 4 - zumindest anfangs - in etwa in der zeitlichen Mitte des Zeitbereichs der elektrischen Diastole 3 gewählt wird. Bereits durch eine im Wesentlichen willkürliche Auswahl des ersten Zeitpunkts 4 innerhalb des Zeitbereichs der elektrischen Diastole 3 kann eine deutliche Verbesserung der Herzratenvariabilität erzielt werden. Dabei hat sich jedoch gezeigt, dass eine weitere Verbesserung möglich ist, bei Durchführung der nachfolgend beschriebenen Schritte einer besonders bevorzugten Ausbildung des gegenständlichen Verfahrens.

Bevorzugt ist vorgesehen, dass aus dem EKG 1 fortwährend ein zeitlicher Verlauf einer Herzrate RR ermittelt wird, daher, dass fortwährend der Effekt der vorgeschlagenen Übung auf den betreffenden Probanden überwacht wird. Fig. 3 zeigt einen beispielhaft dargestellten zeitlichen Verlauf 6 der Herzrate RR. Auf der Abszissenachse ist dabei die Zeit t aufgetragen, und auf der Ordinatenachse die Herzrate RR. Die Nullpunktlage auf der Ordinatenachse ist dabei frei wählbar, und kann etwa als statistisch bekannter Mittelwert einer typischen Herzrate RR vorgegeben werden, relativ zu welcher die beim Probanden gemessenen Herzraten RR eingetragen werden. Die einzelnen Punkte stellen dabei die jeweiligen Messpunkte zu den einzelnen gemessenen Herzraten RR dar.

Nachfolgend ist vorgesehen, den zeitlichen Verlauf 6 der Herzrate RR hinsichtlich Frequenz und/oder Amplitude auszuwerten, wobei unterschiedliche Verfahren im Zeitbereich wie in auch in einem Bildbereich vorgesehen sein können.

Dabei ist bevorzugt vorgesehen, dass aus dem zeitlichen Verlauf 6 der Herzrate RR ein Wert einer Herzraten-Amplitude 9 ermittelt wird. Diese Herzraten-Amplitude 9 ist auch ein Wert der zu diesem Zeitpunkt auftretenden Herzratenvariabilität.

Hiezu ist bevorzugt vorgesehen, in dem zeitlichen Verlauf 6 der Herzrate RR obere Wendepunkte 7 und untere Wendepunkte 8 ermittelt werden, und dass aus jeweils aufeinander folgenden oberen und unteren Wendepunkten 7, 8 der Wert der Herzraten-Amplitude 9 ermittelt wird. Die Ermittlung der oberen Wendepunkte 7 und unteren Wendepunkte 8 kann etwa mit den Mitteln der Kurvendiskussion bzw. der Extremwertaufgaben erfolgen.

Hiezu ist bevorzugt vorgesehen, dass der erste Zeitpunkt 4 des Reizes bzw. der Atemaufforderung innerhalb des Zeitbereichs der elektrischen Diastole 3, zur Ermittlung einer, diesem ersten Zeitpunkt 4 zugeordneten ersten Amplitude, für derart viele aufeinander folgende Herzschläge konstant gehalten wird, bis ein oberer Wendepunkt 7 und ein unterer Wendepunkt 8 der Herzrate RR detektiert wurden. Dabei kann vorgesehen sein, ein Stoppkriterium vorzusehen, falls nach einer unerwartet langen Zeit noch immer keine Herzraten-Amplitude 9 ermittel werden konnte.

Alternativ zur vorstehend beschriebenen Auswertung der Herzraten-Amplitude 9, kann vorgesehen sein, dass ein Teilbereich, insbesondere zwischen zwei gemessenen Herzraten RR, in dem zeitlichen Verlauf 6 der Herzrate RR differenziert wird. Daher, dass zwei gemessenen Herzraten RR mit einer fiktiven geraden verbunden werden, deren Steigung ermittelt wird, und diese Größe als Zielgröße in dem gegenständlichen Regelkreis angesehen wird.

Es ist in Weiterbildung bevorzugt vorgesehen, dass der erste Zeitpunkt 4 des Reizes und/oder der Atemaufforderung innerhalb des Reiz-Zeitbereichs variiert wird, zur Ermittlung eines Zeitpunkts an dem ein zeitlicher Verlauf 6 der Herzrate RR mit vorgebbaren Eigenschaften festgestellt wird. Wie bereits dargelegt, hat sich der Zeitbereich der elektrischen Diastole 3 als besonders vorteilhaft erwiesen, zum Erreichen einer hohen Herzraten-Amplitude 9. Sofern jedoch eine geringe oder mittlere Herzraten-Amplitude 9 erreicht oder verursacht werden soll, sind andere Reiz-Zeitbereiche vorteilhaft.

Wie bereits angedeutet, ist insbesondere vorgesehen, dass der erste Zeitpunkt 4 des Reizes bzw. der Atemaufforderung innerhalb des Zeitbereichs der elektrischen Diastole 3 variiert wird, zur Ermittlung eines Herzraten-Amplituden-Maximum-Zeitpunkts 10 an dem ein Maximum der Herzraten-Amplitude 9 festgestellt wird bzw. vorherrscht, wodurch der Effekt der durchgeführten Übungen noch zusätzlich gesteigert werden kann.

Weiters ist bevorzugt vorgesehen, dass der erste Zeitpunkt 4 innerhalb eines vorgebbaren Rasters variiert wird. Daher ist vorgesehen, dass lediglich eine diskrete und begrenzte Anzahl an Variationen durchgeführt wird, um sowohl schnell eine gutes Ergebnis zu erhalten, wie auch um Endlosschleifen zu vermeiden. Dadurch wird sowohl die Durchführung des Verfahrens unterstützt, wie auch die Implementierbarkeit, insbesondere in mobile Applikationen.

Bei der beschriebenen Ermittlung eines Herzraten-Amplituden-Maximum-Zeitpunkts 10 handelt es sich um ein iteratives Verfahren, wobei vorgesehen ist, nach einer vorgebbaren Anzahl an Variationen abzubrechen, und den zu diesem Punkt bekannten ersten Zeitpunkt 4 mit dem bis dahin höchsten Wert der Herzraten-Amplitude 9 als Herzraten-Amplituden-Maximum-Zeitpunkt 10 festzulegen. Dadurch kann sichergestellt werden, dass tatsächlich ein Herzraten-Amplituden-Maximum-Zeitpunkt 10 ermittelt wird. In Fig. 4 sind beispielhaft sowohl ein erster Zeitpunkt 4, als auch ein Herzraten-Amplituden-Maximum-Zeitpunkts 10 eingetragen.

Sobald der Herzraten-Amplituden-Maximum-Zeitpunkt 10 ermittelt bzw. festgelegt wurde, ist vorgesehen, den Reiz bzw. die Atemaufforderungen an den Herzraten-Amplituden-Maximum-Zeitpunkten 10 zu erzeugen und an die Signaleinheit 5 zu übermitteln, sodass der Proband nunmehr die Atemaufforderungen zu den Zeiten signalisiert bekommt bzw. der Reiz zu den betreffenden Zeiten ausgegeben wird, welche den höchsten Trainingseffekt versprechen.

Zur Anpassung an Veränderungen des Probanden bzw. zur Berücksichtigung einer möglichen Drift des Herzraten-Amplituden-Maximum-Zeitpunkts 10, ist bevorzugt vorgesehen, dass nach einer vorgebbaren Anzahl zum Herzraten-Amplituden-Maximum-Zeitpunkt 10 ausgegebenen Reizen bzw. Atemaufforderungen, der Reiz bzw. die Atemaufforderung in einer vorgebbaren zeitlichen Umgebung um den Herzraten-Amplituden-Maximum-Zeitpunkt 10 vorgebbar variiert wird. Dabei ist bevorzugt vorgesehen, dass die betreffende zeitliche Umgebung eine zeitlich geringe Dauer gegenüber der zeitlichen Dauer der elektrischen Diastole 3 aufweist.

In Weiterbildung ist dabei vorgesehen, dass der Herzraten-Amplituden-Maximum-Zeitpunkt 10 bei Ermittlung eines neuen Zeitpunkts eines Maximums der Herzraten-Amplitude 9 an diesen neuen Zeitpunkt angepasst wird. Dadurch kann eine stabile und effektive Trainingsdurchführung über einen längeren Zeitraum hinweg sichergestellt werden.

## Patentansprüche

1. Vorrichtung (11) zur Erstellung und Ausgabe eines Reizes und/oder einer Atemaufforderung für einen Menschen und/oder ein Tier, wobei die Vorrichtung (11) eine Herzsignalermittlungseinheit, eine Auswerteeinheit (2) und eine Signaleinheit (5) aufweist, wobei die Herzsignalermittlungseinheit mit Signaleingängen sowie mit der Auswerteeinheit (2) verbunden ist, und wobei die Auswerteeinheit (2) mit der Signaleinheit (5) verbunden ist, **dadurch gekennzeichnet, dass** die Auswerteeinheit (2) ausgebildet ist innerhalb eines, von der Herzsignalermittlungseinheit aufgenommenen Herzschlagsignals des Menschen oder Tieres einen vorgebbarer Reiz-Zeitbereich zwischen zwei Herzschlägen zu ermitteln, wobei der vorgebbare Reiz-Zeitbereich ein Zeitbereich der elektrischen Diastole (3) ist, dass die Auswerteeinheit (2) weiters ausgebildet ist einen ersten Reiz und/oder eine erste Atemaufforderung an einem ersten Zeitpunkt (4) innerhalb des Reiz-Zeitbereichs eines vorgebbaren Start-Herzschlages zu erzeugen und zur Ausgabe an die Signaleinheit (5) zu übermitteln.

2. Vorrichtung (11) nach Anspruch 1, **dadurch gekennzeichnet, dass** die Herzsignalermittlungseinheit eine EKG-Einheit (13) ist.

3. Vorrichtung (11) nach einem der Ansprüche 1 oder 2, **dadurch gekennzeichnet, dass** die Atemaufforderung eine Einatemaufforderung und/oder eine Ausatemaufforderung ist, oder dass der, als Signal ausgegebene Reiz ein optischer Reiz ist, und dass das Signal als optisches Signal ausgegeben wird, oder dass der, als Signal ausgegebene Reiz ein akustischer Reiz ist, und dass das Signal als akustisches Signal ausgegeben wird, oder dass der, als Signal ausgegebene Reiz ein taktiler Reiz ist, und dass das Signal als mechanisches Signal ausgegeben wird, oder dass der, als Signal ausgegebene Reiz ein olfaktorischer Reiz ist, und dass das Signal als Freisetzung wenigstens eines Duftstoffes ausgegeben wird.

4. Vorrichtung (11) nach einem der Ansprüche 1 bis 3, **dadurch gekennzeichnet, dass** die Auswerteeinheit (2) weiters dazu ausgebildet ist einen zweiten Reiz und/oder eine zweite Atemaufforderung an dem ersten Zeitpunkt (4) innerhalb des Reiz-Zeitbereichs eines Herzschlages zu erzeugen und an die Signaleinheit (5) zu übermitteln, welcher Herzschlag eine vorgebbare Anzahl Herzschläge nach dem ersten Herzschlag erfolgt.

5. Vorrichtung (11) nach Anspruch 4, **dadurch gekennzeichnet, dass** der zweite Reiz und/oder die zweite Atemaufforderung unterschiedlich von dem ersten Reiz bzw. der ersten Atemaufforderung ist.

6. Vorrichtung (11) nach einem der Ansprüche 1 bis 5, **dadurch gekennzeichnet, dass** die Auswerteeinheit (2) weiters dazu ausgebildet ist aus dem Herzschlagsignal, insbesondere dem EKG (1), fortwährend einen zeitlichen Verlauf (6) einer Herzrate (RR) zu ermitteln, den zeitlichen Verlauf (6) der Herzrate (RR) hinsichtlich Frequenz und Amplitude auszuwerten, und vorzugsweise aus dem zeitlichen Verlauf (6) der Herzrate (RR) einen Wert einer Herzraten-Amplitude (9) zu ermitteln.

7. Vorrichtung (11) nach Anspruch 6, **dadurch gekennzeichnet, dass** die Auswerteeinheit (2) weiters dazu ausgebildet ist in dem zeitlichen Verlauf (6) der Herzrate (RR) obere Wendepunkte (7) und untere Wendepunkte (8) zu ermitteln, und aus jeweils aufeinander folgenden oberen und unteren Wendepunkten (7, 8) den Wert der Herzraten-Amplitude (9) zu ermittel.

8. Vorrichtung (11) nach Anspruch 7, **dadurch gekennzeichnet, dass** die Auswerteeinheit (2) weiters dazu ausgebildet ist den ersten Zeitpunkt (4) des Reizes und/oder der Atemaufforderung innerhalb des Reiz- Zeitbereichs für derart viele aufeinander folgende Herzschläge konstant zu halten, bis ein oberer Wendepunkt (7) und ein unterer Wendepunkt (8) der Herzrate detektiert wurden, wobei vorzugsweise ein Teilbereich, insbesondere zwischen zwei gemessenen Herzraten (RR), in dem zeitlichen Verlauf (6) der Herzrate (RR) differenziert wird.

9. Vorrichtung (11) nach einem der Ansprüche 1 bis 8, **dadurch gekennzeichnet, dass** die Vorrichtung weiters einen Brustgurt aufweist, welcher mit Messgeräten zur Aufnahme einer Kontraktion und Expansion eines Brustkorbes ausgestattet ist, und welcher mit der Auswerteinheit (2) in Kommunikationsverbindung steht, und dass die Auswerteeinheit (2) weiters dazu ausgebildet ist bei Ausgabe einer Atemaufforderung zu überprüfen, ob und/oder nach welcher Atemlatenz-Zeit nach der Abgabe der Atemaufforderung der Mensch und/oder das Tier atmet, und den ersten Zeitpunkt (4) um die Atemlatenz-Zeit zu korrigieren.

10. Vorrichtung (11) nach einem der Ansprüche 6 bis 9, **dadurch gekennzeichnet, dass** die Auswerteeinheit (2) weiters dazu ausgebildet ist den ersten Zeitpunkt (4) des Reizes und/oder der Atemaufforderung innerhalb des Reiz-Zeitbereichs zu variieren, und weiters einen Zeitpunkt zu ermitteln an dem ein zeitlicher Verlauf (6) der Herzrate (RR) mit vorgebbaren Eigenschaften festgestellt wird.

11. Vorrichtung (11) nach Anspruch 10, **dadurch gekennzeichnet, dass** die Auswerteeinheit (2) weiters dazu ausgebildet ist den ersten Zeitpunkt (4), vorzugsweise innerhalb eines vorgebbaren Rasters, zu variieren, zur Ermittlung eines Herzraten-Amplituden-Maximum-Zeitpunkts (10) an dem ein Maximum der Herzraten-Amplitude (9) festgestellt wird.

12. Vorrichtung (11) nach Anspruch 11, **dadurch gekennzeichnet, dass** die Auswerteeinheit (2) weiters dazu ausgebildet ist nach Ermittlung des Herzraten-Amplituden-Maximum-Zeitpunkts (10) den Reiz und/oder die Atemaufforderungen an den Herzraten-Amplituden-Maximum-Zeitpunkten (10) zu erzeugen und an die Signaleinheit zu übermitteln.

13. Vorrichtung (11) nach Anspruch 11 oder 12, **dadurch gekennzeichnet, dass** die Auswerteeinheit (2) weiters dazu ausgebildet ist nach einer vorgebbaren Anzahl zum Herzraten-Amplituden-Maximum-Zeitpunkt (10) ausgegebenen Reizen und/oder Atemaufforderungen, den Reiz und/oder die Atemaufforderung in einer vorgebbaren zeitlichen Umgebung um den Herzraten-Amplituden-Maximum-Zeitpunkt (10) vorgebbar zu variieren.

14. Vorrichtung (11) nach einem der Ansprüche 11 bis 13, **dadurch gekennzeichnet, dass** die Auswerteeinheit (2) weiters dazu ausgebildet ist den Herzraten-Amplituden-Maximum-Zeitpunkt (10) bei Ermittlung eines neuen Zeitpunkts eines Maximums der Herzraten-Amplitude (9) an diesen neuen Zeitpunkt anzupassen.

## Claims

1. Device (11) for generating and outputting a stimulus and/or an instruction to breathe for a human being and/or an animal, wherein the device (11) comprises a heart signal detection unit, an evaluation unit (2) and a signal unit (5), wherein the heart signal detection unit is connected to signal inputs and to the evaluation unit (2), and wherein the evaluation unit (2) is connected to the signal unit (5), **characterized in that** the evaluation unit (2) is designed to determine, within a heartbeat signal of the human being or animal recorded by the heart signal detection unit, a predeterminable stimulation time range between two heartbeats, wherein the predeterminable stimulation time range is a time range of the electrical diastole (3), **in that** the evaluation unit (2) is further designed to generate a first stimulus and/or a first instruction to breathe at a first time (4) within the stimulation time range of a predeterminable starting heartbeat and to transmit it to the signal unit (5) for output.

2. Device (11) according to claim 1, **characterized in that** the heart signal detection unit is an ECG unit (13).

3. Device (11) according to one of the claims 1 or 2, **characterized in that** the instruction to breathe is an inhalation instruction and/or an exhalation instruction, or **in that** the stimulus output as a signal is an optical stimulus, and **in that** the signal is output as an optical signal, or **in that** the stimulus output as a signal is an acoustic stimulus, and **in that** the signal is output as an acoustic signal, or **in that** the stimulus output as a signal is a tactile stimulus, and **in that** the signal is output as a mechanical signal, or **in that** the stimulus output as a signal is an olfactory stimulus, and **in that** the signal is output as a release of at least one odorant.

4. Device (11) according to one of the claims 1 to 3, **characterized in that** the evaluation unit (2) is further designed to generate a second stimulus and/or a second instruction to breathe at the first time (4) within the stimulation time range of a heartbeat and to transmit it to the signal unit (5), which heartbeat occurs a predeterminable number of heartbeats after the first heartbeat.

5. Device (11) according to claim 4, **characterized in that** the second stimulus and/or the second instruction to breathe is different from the first stimulus and/or the first instruction to breathe.

6. Device (11) according to one of claims 1 to 5, **characterized in that** the evaluation unit (2) is further designed to continuously determine a time course (6) of a heart rate (RR) from the heartbeat signal, in particular the ECG (1), to evaluate the time course (6) of the heart rate (RR) with regard to frequency and amplitude, and preferably to determine a value of a heart rate amplitude (9) from the time course (6) of the heart rate (RR).

7. Device (11) according to claim 6, **characterized in that** the evaluation unit (2) is further designed to determine upper turning points (7) and lower turning points (8) in the time course (6) of the heart rate (RR), and to determine the value of the heart rate amplitude (9) from successive upper and lower turning points (7, 8) in each case.

8. Device (11) according to claim 7, **characterized in that** the evaluation unit (2) is further designed to keep the first time (4) of the stimulus and/or the instruction to breathe within the stimulation time range constant for such a large number of successive heartbeats until an upper turning point (7) and a lower turning point (8) of the heart rate have been detected, wherein preferably a subrange, in particular between two measured heart rates (RR), are differentiated in the time course (6) of the heart rate (RR).

9. Device (11) according to one of the claims 1 to 8, **characterized in that** the device further has a chest belt which is equipped with measuring devices for recording a contraction and expansion of a thorax, and which is in communication connection with the evaluation unit (2), and **in that** the evaluation unit (2) is further designed, when an instruction to breathe is issued, to check whether and/or after which respiratory latency time the human being and/or the animal is breathing after the issue of the instruction to breathe, and to correct the first time (4) by the respiratory latency time.

10. Device (11) according to one of the claims 6 to 9, **characterized in that** the evaluation unit (2) is further designed to vary the first time (4) of the stimulus and/or of the instruction to breathe within the stimulation time range, and further to determine a time at which a time course (6) of the heart rate (RR) with predeterminable properties is determined.

11. Device (11) according to claim 10, **characterized in that** the evaluation unit (2) is further designed to vary the first time (4), preferably within a predeterminable grid, in order to determine a heart rate amplitude maximum time (10) at which a maximum of the heart rate amplitude (9) is detected.

12. Device (11) according to claim 11, **characterized in that** the evaluation unit (2) is further designed to generate the stimulus and/or the instructions to breathe at the heart rate amplitude maximum times (10) after determination of the heart rate amplitude maximum time (10) and to transmit them to the signal unit.

13. Device (11) according to claim 11 or 12, **characterized in that** the evaluation unit (2) is further designed, after a predeterminable number of stimuli and/or instructions to breathe that are output at the heart rate amplitude maximum time (10), to vary in a predeterminable manner the stimulus and/or the instruction to breathe in a predeterminable time environment around the heart rate amplitude maximum time (10).

14. Device (11) according to one of claims 11 to 13, **characterized in that** the evaluation unit (2) is further designed to adapt the heart rate amplitude maximum time (10) to this new time when a new time of a maximum of the heart rate amplitude (9) is determined.

## Revendications

1. Dispositif (11) pour générer et émettre un stimulus et/ou une commande respiratoire pour un sujet humain et/ou un animal, lequel dispositif (11) comporte une unité de détection de signal cardiaque, une unité d'analyse (2) et une unité de signalisation (5), l'unité de détection de signal cardiaque étant reliée à des entrées de signal et à l'unité d'analyse (2) et l'unité d'analyse (2) étant reliée à l'unité de signalisation (5), **caractérisé en ce que** l'unité d'analyse (2) est conçue pour détecter dans un signal de battement cardiaque du sujet humain ou de l'animal capté par l'unité de détection de signal cardiaque un intervalle de temps de stimulation pouvant être prédéterminé entre deux battements cardiaques, lequel intervalle de temps de stimulation pouvant être prédéterminé est un intervalle de temps de la diastole électrique (3), **en ce que** l'unité d'analyse (2) est en outre conçue pour générer un premier stimulus et/ou une première commande respiratoire à un premier instant (4) dans l'intervalle de temps de stimulation d'un battement de cœur de départ pouvant être prédéterminé et les transmettre à l'unité de signalisation (5) en vue de son émission en sortie.

2. Dispositif (11) selon la revendication 1, **caractérisé en ce que** l'unité de détection de signal cardiaque est une unité d'ECG (13).

3. Dispositif (11) selon l'une des revendications 1 ou 2, **caractérisé en ce que** la commande respiratoire est une commande d'inspiration et/ou une commande d'expiration ou **en ce que** le stimulus émis comme signal est un stimulus optique et **en ce que** le signal est émis sous forme de signal optique, ou **en ce que** le stimulus émis comme signal est un stimulus acoustique et **en ce que** le signal est émis sous forme de signal acoustique, ou **en ce que** le stimulus émis comme signal est un stimulus tactile et **en ce que** le signal est émis sous forme de signal mécanique, ou **en ce que** le stimulus émis comme signal est un stimulus olfactif et **en ce que** le signal est émis sous forme de la libération d'au moins une substance odorante.

4. Dispositif (11) selon l'une des revendications 1 à 3, **caractérisé en ce que** l'unité d'analyse (2) est conçue en outre pour générer un deuxième stimulus et/ou une deuxième commande respiratoire au premier instant (4) dans l'intervalle de temps de stimulation d'un battement cardiaque et les transmettre à l'unité de signalisation (5), ce battement cardiaque survenant à un nombre pouvant être prédéterminé de battements après le premier battement cardiaque.

5. Dispositif (11) selon la revendication 4, **caractérisé en ce que** le deuxième stimulus et/ou la deuxième commande respiratoire sont différents du premier stimulus et/ou de la première commande respiratoire.

6. Dispositif (11) selon l'une des revendications 1 à 5, **caractérisé en ce que** l'unité d'analyse (2) est en outre conçue pour déterminer en continu, à partir du signal de battement cardiaque, en particulier de l'ECG (1), une évolution dans le temps (6) d'une fréquence cardiaque (RR), pour analyser l'évolution dans le temps (6) de la fréquence cardiaque (RR) en termes de fréquence et d'amplitude et, de préférence, pour déterminer à partir de l'évolution dans le temps (6) de la fréquence cardiaque (RR) une valeur d'amplitude de la fréquence cardiaque (9).

7. Dispositif (11) selon la revendication 6, **caractérisé en ce que** l'unité d'analyse (2) est en outre conçue pour déterminer, dans l'évolution dans le temps (6) de la fréquence cardiaque (RR), des points de retournement hauts (7) et des points de retournement bas (8) et pour déterminer, à partir de points de retournement hauts et bas (7, 8) successifs, la valeur de l'amplitude de la fréquence cardiaque (9).

8. Dispositif (11) selon la revendication 7, **caractérisé en ce que** l'unité d'analyse (2) est en outre conçue pour garder le premier instant (4) du stimulus et/ou de la commande respiratoire constant dans l'intervalle de temps de stimulation pendant un nombre suffisant de battements de cœur successifs pour qu'un point de retournement haut (7) et un point de retournement bas (8) de la fréquence cardiaque soient détectés, en différenciant de préférence une plage partielle, en particulier entre deux fréquences cardiaques (RR) mesurées, dans l'évolution dans le temps (6) de la fréquence cardiaque (RR).

9. Dispositif (11) selon l'une des revendications 1 à 8, **caractérisé en ce qu'**il comprend en outre une sangle thoracique équipée d'appareils de mesure pour capter une contraction et une expansion d'une cage thoracique et qui communique avec l'unité d'analyse (2), et **en ce que** l'unité d'analyse (2) est en outre conçue pour vérifier, lors de l'émission d'une commande respiratoire, si et/ou après quel temps de latence suivant l'émission de la commande respiratoire le sujet humain et/ou l'animal respire et pour corriger le premier instant (4) avec le temps de latence respiratoire.

10. Dispositif (11) selon l'une des revendications 6 à 9, **caractérisé en ce que** l'unité d'analyse (2) est en outre conçue pour faire varier le premier instant (4) du stimulus et/ou de la commande respiratoire dans l'intervalle de temps de stimulation et pour déterminer en outre un instant auquel une évolution dans le temps (6) de la fréquence cardiaque (RR) ayant des propriétés pouvant être prédéterminées est constatée.

11. Dispositif (11) selon la revendication 10, **caractérisé en ce que** l'unité d'analyse (2) est en outre conçue pour faire varier le premier instant (4), de préférence selon une grille pouvant être prédéterminée, pour la détermination d'un instant d'amplitude maximale de la fréquence cardiaque (10) auquel un maximum de l'amplitude de la fréquence cardiaque (9) est constaté.

12. Dispositif (11) selon la revendication 11, **caractérisé en ce que** l'unité d'analyse (2) est en outre conçue pour générer, après la détermination de l'instant d'amplitude maximale de la fréquence cardiaque (10), le stimulus et/ou les commandes respiratoires aux instants d'amplitude maximale de la fréquence cardiaque (10) et les transmettre à l'unité de signalisation.

13. Dispositif (11) selon la revendication 11 ou 12, **caractérisé en ce que** l'unité d'analyse (2) est en outre conçue pour, après un nombre pouvant être prédéterminé de stimuli et/ou de commandes respiratoires émis à l'instant d'amplitude maximale de la fréquence cardiaque (10), faire varier le stimulus et/ou la commande respiratoire de façon pouvant être prédéterminée dans un environnement temporel pouvant être prédéterminé autour de l'instant d'amplitude maximale de la fréquence cardiaque (10).

14. Dispositif (11) selon l'une des revendications 11 à 13, **caractérisé en ce que** l'unité d'analyse (2) est en outre conçue pour adapter l'instant d'amplitude maximale de la fréquence cardiaque (10) à un nouvel instant de maximum de l'amplitude de la fréquence cardiaque (9) lorsque ce nouvel instant est détecté.
